# FASCICULE DE BREVET EUROPEEN

(11) **EP 1 301 534 B1**
(45) Date de publication et mention de la délivrance du brevet: **10.01.2007**
(21) Numéro de dépôt: 01955438.5
(22) Date de dépôt: 18.07.2001
(51) Int. Cl.: C07K 14/52, A61K 38/19, A61P 35/00, C12N 15/11

(54) **PF-4 MUTE, SES FRAGMENTS ET PEPTIDES DE FUSION MUTES, LEURS ANALOGUES, LES SEQUENCES D'ADN, ADNc ET ARNm CORRESPONDANTES, ET LEUR UTILISATION DANS L'INHIBITION DE L'ANGIOGENESE**
MUTIERTES PF4, SEINE MUTIERTE FRAGMENTE UND FUSIONSPEPTIDE, DEREN ANALOGA, ZUGEHÖRIGE DNA-, CDNA- UND MRNA-SEQUENZEN UND DEREN VERWENDUNG ZUR HEMMUNG DER ANGIOGENESE
MUTATED PF-4, ITS FRAGMENTS AND MUTATED FUSION PEPTIDES, THEIR ANALOGUES, CORRESPONDING DNA, CDNA AND MRNA SEQUENCES AND THEIR USE FOR INHIBITING ANGIOGENESIS

(30) Priorité: 19.07.2000 FR 0009464
(43) Date de publication de la demande: 16.04.2003
(73) Titulaire: UNIVERSITE DE BORDEAUX I, F-33405 Talence Cedex (FR); INSTITUT DES VAISSEAUX ET DU SANG, 75475 Paris Cedex 10 (FR)
(72) Inventeur: BIKFALVI, Andreas, F-33170 Gradignan (FR); ALEMANY, Monica, F-75020 Paris (FR)
(74) Mandataire: Vercaemer, Laurence
(86) Numéro de dépôt international: PCT/FR2001/002341
(87) Numéro de publication internationale: WO 2002/006300

(56) Documents cités:
- EP-A- 0 378 364
- WO-A-97/12036
- WO-A-99/41283
- FR-A- 2 691 153
- JOUAN VALERIE ET AL: "Inhibition of in vitro angiogenesis by platelet factor-4-derived peptides and mechanism of action." BLOOD, vol. 94, no. 3, 1 août 1999 (1999-08-01), pages 984-993, XP002168845 ISSN: 0006-4971
- LECOMTE-RACLET E A: "New insights into the negative regulation of hematopoiesis by chemokine platelet factor 4 and related peptides" BLOOD,W.B. SAUNDERS, PHILADELPHIA, VA,US, vol. 91, no. 8, 15 avril 1998 (1998-04-15), pages 2772-2780, XP002148262 ISSN: 0006-4971
- MAIONE T E ET AL: "INHIBITION OF ANGIOGENESIS BY RECOMBINANT HUMAN PLATELET FACTOR-4 AND RELATED PEPTIDES" SCIENCE,US,AMERICAN ASSOCIATION FOR THE ADVANCEMENT OF SCIENCE,, vol. 247, no. 49, 5 janvier 1990 (1990-01-05), pages 77-79, XP002027204 ISSN: 0036-8075
- WALZ A ET AL: "EFFECTS OF THE NEUTROPHIL-ACTIVATING PEPTIDE NAP-2, PLATELET BASIC PROTEIN, CONNECTIVE TISSUE-ACTIVATING PEPTIDE III, AND PLATELET FACTOR 4 ON HUMAN NEUTROPHILS" JOURNAL OF EXPERIMENTAL MEDICINE,TOKYO,JP, vol. 170, 1 novembre 1989 (1989-11-01), pages 1745-1750, XP000943072 ISSN: 0022-1007

## Description

La présente invention a pour objet un peptide correspondant au facteur plaquettaire 4 (PF-4) muté, ou à ses fragments et peptides de fusion mutés, ou à leurs analogues. Elle a également pour objet les séquences correspondantes d'ADN et ADNc et ARNm, et l'utilisation de ces peptides et séquences dans l'inhibition de l'angiogenèse. Plus particulièrement, l'invention a pour objet un PF-4 portant une mutation en position 56, la glutamine (Q) étant remplacée par un acide aminé basique, tel que l'arginine (R), la lysine (K) ou l'histidine (H), ou un fragment ou peptide de fusion dérivé du PF-4 et comprenant cette mutation, ou leurs analogues.

Le PF-4 est une protéine connue, dont on a séquencé les 70 acides aminés (séquence 1-70, Voir SEQ. ID. No : 1).

On a montré que le PF-4 natif présente une certaine activité inhibitrice in vitro et in vivo de l'angiogenèse et de la prolifération des cellules endothéliales, et peut donc être utilisé dans le traitement de l'angiogenèse et par conséquent des pathologies qui y sont liées, telles que par exemple les cancers, tumeurs et maladies inflammatoires chroniques.

Le mode d'action du PF-4 est encore imparfaitement connu. Néanmoins, on a montré qu'il inhibe la fixation du FGF-2 (Fibroblast Growth Factor) et du VEGF (Vascular Endothelial Growth Factor) à leurs récepteurs, et la fixation du FGF-2 aux protéoglycannes. Par ailleurs, le PF-4 inhibe également la dimérisation du FGF-2.

On cherche cependant à augmenter l'efficacité des traitements de l'angiogenèse, et en particulier à augmenter l'activité des inhibiteurs, afin de pouvoir soit limiter la quantité d'inhibiteur utilisée au cours du traitement en conservant constante l'activité inhibitrice, soit obtenir une plus forte activité inhibitrice avec la même quantité d'inhibiteur.

On a donc cherché à obtenir à partir du PF-4 des peptides ayant une activité plus importante que celle du PF-4 natif. Plusieurs méthodes ont été mises en oeuvre.

Une voie de recherche a consisté à conjuguer un PF-4 (ou un fragment ou mutant de celui-ci) avec une autre entité permettant d'augmenter l'activité inhibitrice du PF-4.

En particulier, le brevet EP 723 015 décrit l'utilisation d'un conjugué polypeptidique comprenant le PF-4 ou un fragment ou mutant de celui-ci, conjugué à une seconde molécule choisie parmi les fluorophores, les chélatants, les molécules porteuses, les anticorps, les toxines et les molécules de récepteurs cellulaires ou leurs ligands complémentaires. Dans ce même document, il est précisé qu'un peptide de 13 acides aminés correspondant à la séquence C-terminale du PF-4 dans laquelle les deux couples de lysine ont été remplacés par deux couples acide glutamique - glutamine, présente une activité inhibitrice de l'angiogenèse.

Les demandes de brevet WO93/02192 et WO95/1241 décrivent également la conjugaison du PF-4 et d'une autre entité.

Une autre voie de recherche a consisté à dériver du PF-4 natif des polypeptides plus actifs que ce PF-4.

La demande de brevet EP 378 364 décrit des peptides et fragments dérivés du PF-4. En particulier, elle décrit une protéine d'environ 71 acides aminés comprenant les acides aminés 1 à 60 du PF-4 natif, les acides aminés 61 et suivants (extrémité C-terminale) pouvant correspondre à différentes séquences. L'intérêt de l'activité inhibitrice de la séquence de 13 acides aminés correspondant à l'extrémité C-terminale du PF-4 natif est particulièrement souligné.

Le document WO 99/41283 décrit des fragments de PF-4.

La demande de brevet EP 176 588 concerne différents polypeptides de 15 à 80 acides aminés présentant un certain nombre de mutations par rapport à la séquence du PF-4. De très nombreuses séquences sont couvertes par les formules données, toutes ces séquences ayant une extrémité N-terminale acide (c'est-à-dire présence d'acide glutamique ou aspartique parmi les 6 à 15 acides aminés N-terminaux), et une extrémité C-terminale basique (c'est-à-dire présence de lysine ou arginine parmi les 6 à 15 acides aminés C-terminaux).

Contrairement à l'enseignement de l'art antérieur, qui implique une conjugaison avec une deuxième molécule ou de nombreuses mutations par rapport à la séquence du PF-4 natif, les Demandeurs ont constaté avec surprise qu'une unique mutation au niveau de l'acide aminé 56 de la séquence du PF-4 ou d'un analogue ou fragment ou peptide de fusion dérivé du PF-4 et comprenant cet acide aminé, permet d'augmenter considérablement l'activité inhibitrice de l'angiogenèse du peptide obtenu.

L'invention a donc pour objet un PF-4 ou ses analogues dans lequel la glutamine (Q) en position 56 est remplacée par un acide aminé basique. Cet acide aminé basique peut être l'arginine (R) (Voir SEQ. ID. No : 2), la lysine (K) ou l'histidine (H). Il s'agira de préférence de l'arginine. L'invention a également pour objet un fragment ou un peptide de fusion dérivé du PF-4, ou un de leurs analogues, comprenant cette mutation.

Le terme « peptide PF-4 selon l'invention» désignera l'ensemble de ces peptides, à savoir le PF-4, ses analogues, les fragments et peptides de fusion dérivés du PF-4 et leurs analogues, comprenant cette mutation particulière au niveau de l'acide aminé correspondant à la glutamine 56 dans la séquence du PF-4 natif.

Un peptide PF-4 non muté sera dénommé « DLQ ». Un peptide PF-4 comprenant la mutation en position 56, c'est-à-dire un peptide PF-4 selon l'invention, sera dénommé « DLR ».

Le terme « analogues » désigne des peptides dont les séquences présentent les unes par rapport aux autres des mutations n'ayant pas de conséquence néfaste sur l'activité du peptide. Dans la présente demande, les analogues auront tous une activité inhibitrice de l'angiogenèse. Les mutations peuvent consister en particulier en le remplacement d'un acide aminé par un acide aminé du même type, comme par exemple le remplacement d'une valine (V) par une alanine (A) qui sont tous les deux des acides aminés aliphatiques, ou le remplacement d'une thréonine (T) par un sérine (S) qui sont tous les deux des acides aminés aliphatiques oxysubstitués.

Un fragment muté selon l'invention pourra correspondre en particulier au fragment muté 47-70 (Voir SEQ. ID. No : 3).

Tout fragment dérivé du PF-4 natif présentant une activité inhibitrice de l'angiogenèse fait également partie des fragments selon l'invention, quelle que soit sa longueur, à la condition qu'il comprenne cette mutation particulière au niveau de l'acide aminé correspondant à la glutamine 56 dans la séquence du PF-4 natif. Il peut s'agir d'un fragment présentant par rapport au PF-4 des acides aminés en moins et/ou des acides aminés en plus (un exemple est le fragment 47-70).

Un peptide de fusion muté selon l'invention pourra correspondre en particulier au peptide de fusion 17-34/47-70 (Voir SEQ. ID. No : 4). Des essais ont montré que ce peptide de fusion présente une activité inhibitrice supérieure à celle du PF-4 natif ou du fragment 47-70. Un tel peptide de fusion muté sera donc particulièrement intéressant.

Tout peptide de fusion issu du PF-4 natif présentant une activité inhibitrice de l'angiogenèse fait également partie des peptides de fusion selon l'invention, à la condition qu'il comprenne cette mutation particulière au niveau de l'acide aminé correspondant à la glutamine 56 dans la séquence du PF-4 natif.

Le numéro 56 est donné à l'acide aminé muté par référence à sa position dans la séquence de PF-4 natif. Il est évident que cet acide aminé ne sera pas nécessairement à la même position 56 dans tous les peptides PF-4 selon l'invention. Par exemple, dans le fragment 47-70, l'acide aminé portant la mutation en 56 est en fait à la position 10. On utilisera néanmoins le terme « mutation en 56 » quelle que soit la position réelle de l'acide aminé dans la séquence du peptide.

Un peptide PF-4 selon l'invention présentera une I₅₀ (concentration en peptide permettant d'obtenir une inhibition de 50%) inférieure à la valeur de I₅₀ du même peptide non muté en 56.

De préférence, l'I₅₀ d'un peptide PF-4 selon l'invention sera de 2 à 20 inférieure, de préférence de 4 à 15 fois inférieure, de préférence encore de 5 à 10 fois inférieure à la valeur d'I₅₀ du même peptide ne présentant pas la mutation, c'est-à-dire du peptide de séquence identique sauf pour la mutation en 56.

L'I₅₀ peut être mesurée par des techniques classiques connues de l'homme du métier, comme celle décrite dans Jouan V., Canron X., Alemany M., Caen J., Quentin G., Plouet J., and Bikfalvi A. (1999), Modulation of in vitro angiogenesis by Platelet factor-4 derived peptides and mechanism of action, *Blood,* 94 : 984-93, ou dans Perollet C., Han Z.C., Savona C., Caen J.P., et Bikfalvi A. (1998) Platelet Factor-4 modulates Fibroblast Growth Factor-2 Activity and inhibits FGF-2 dimerization, *Blood* 91 : 1-12.

Les peptides PF-4 selon l'invention pourront être obtenus par les techniques habituelles connues de l'homme du métier, à savoir notamment à partir du PF-4 natif (clivage enzymatique), par synthèse, par des techniques recombinantes, par chimie combinatoire.

L'invention a également pour objet une composition pharmaceutique comprenant l'un des peptides PF-4 selon l'invention, en combinaison avec un ou plusieurs excipients acceptables pharmaceutiquement. Cette composition pourra être utilisée pour le traitement ou la prévention des maladies liées à l'angiogenèse, telles que le cancer, les maladies vasculaires de la rétine (rétinopathies), les maladies inflammatoires chroniques (comme la polyarthrite rhumatoide chronique), les angiomes, les hémangiomes et certaines hémopathies (leucémies). Elle pourra être administrée par exemple par intraveineuse ou en sous-cutané.

L'invention a également pour objet les séquences d'ADN, d'ADNc ou d'ARNm codant pour les peptides PF-4 selon l'invention, obtenues selon les techniques connues de l'homme du métier.

L'invention a également pour objet l'utilisation des peptides PF-4 selon l'invention et/ou des séquences d'ADN, ADNc ou ARNm pour la préparation d'un médicament pour le traitement ou la prévention des maladies liées à l'angiogenèse. En effet, compte tenu des résultats obtenus in vitro, il est attendu que seront également inhibées l'angiogenèse in vivo (par exemple sur cornée de lapin) de même que l'angiogenèse ex vivo (par exemple inhibition de l'angiogenèse sur membrane chorioallantoidienne). Un tel médicament pourra être administré par exemple en intraveineuse ou en sous-cutané.

### EXEMPLES

Des essais ont été effectués afin d'évaluer l'activité inhibitrice d'un peptide 47-70 muté par rapport au peptide 47-70 contrôle. La mutation en 56 correspond ici à l'arginine.

Plus particulièrement, on a évalué l'effet inhibiteur de chaque peptide en testant l'effet inhibiteur sur la liaison du FGF-2 ou du VEGF à leurs récepteurs de haute affinité (Tableau 1), la liaison du FGF-2 aux protéoglycannes (Tableau 2), et la prolifération cellulaire (Tableau 3).

L'inhibition de la liaison du FGF-2 aux récepteurs de haute affinité (Tableau 1) est mesurée de la façon suivante. Le FGF-2 est radiomarqué à ¹²⁵I-Na, et 10 ng/ml sont incubés avec les cellules endothéliales pendant 2h à 4°C. La radioactivité liée aux récepteurs de haute affinité est ensuite déterminée. La liaison spécifique est estimée par soustraction de la liaison non-spécifique de la liaison totale (en présence d'un excès de 100 fois de ligand froid par rapport au ligand chaud). La I₅₀ est la concentration qui inhibe la liaison spécifique de 50 %.

L'inhibition de la liaison du VEGF aux récepteurs de surface (Tableau 1) est mesurée de la façon suivante. Le VEGF est radiomarqué à ¹²⁵I-Na et 10 ng/ml sont incubés avec les cellules endothéliales pour 2 h à 4°C. La radioactivité liée aux récepteurs est ensuite déterminée. La liaison spécifique est estimée par soustraction de la liaison non-spécifique de la liaison totale (en présence d'un excès de 100 fois de ligand froid par rapport au ligand chaud). La I₅₀ est la concentration qui inhibe la liaison spécifique de 50 %.

L'inhibition de la liaison du FGF-2 aux protéoglycanes (Tableau 2) est mesurée de la façon suivante. Le FGF-2 est radiomarqué à ¹²⁵I-Na, et 10 ng/ml sont incubés avec les cellules endothéliales pendant 2h à 4°C. La radioactivité liée aux protéoglycanes est ensuite déterminée. La liaison spécifique est estimée par soustraction de la liaison non-spécifique de la liaison totale (en présence d'un excès de 100 fois de ligand froid par rapport au ligand chaud). La I₅₀ est la concentration qui inhibe la liaison spécifique de 50 %.

L' inhibition de l'activité biologique du FGF-2 (Tableau 3) est mesurée de la façon suivante : 20 000 cellules sont incubées avec 10 ng/ml de FGF-2 en milieu DMEM contenant 1 % de sérum de veau foetal (SVF) dans des boîtes de 3,5 cm de diamètre en présence ou en absence de peptide. Les cellules sont comptées au 6^{ème} jour en utilisant un compteur Coulter. Les expériences sont fait en double. La I₅₀ est la concentration qui inhibe la prolifération induite par le FGF-2 de 50 %.

On pourra trouver une description plus détaillée de ces mesures de I₅₀ dans Jouan et al. et dans Perollet et al. cités plus haut.

On compare les activités du peptide 47-70 DLR, c'est-à-dire du peptide muté en position 56, à celle du peptide 47-70, non muté, à titre d'exemple comparatif.

**TABLEAU 1 :**

| Valeurs de I₅₀ correspondant à l'inhibition de la liaison du FGF-2 ou du VEGF aux récepteurs de haute affinité par chaque peptide : | | |
|---|---|---|
| | Liaison du FGF-2 | Liaison du VEGF |
| Peptide 47-70 DLQ | 1,5 µM | 1, 5 µM |
| Peptide 47-70 DLR | 0,3 µM | 0,3 µM |

**TABLEAU 2 :**

| Valeurs de I₅₀ correspondant à l'inhibition de la liaison du FGF-2 aux protéoglycannes par chaque peptide : | |
|---|---|
| Peptide 47-70 DLQ | 3,5 µM |
| Peptide 47-70 DLR | 0,6 µM |

**TABLEAU 3 :**

| Valeurs de I₅₀ correspondant à l'inhibition de la prolifération cellulaire par chaque peptide : | |
|---|---|
| Peptide 47-70 DLQ | 2,8 µM |
| Peptide 47-70 DLR | 0,4 µM |

On peut constater dans chaque cas que le peptide muté présente des valeurs de I₅₀ très largement inférieures aux valeurs de I₅₀ pour le peptide contrôle. En d'autres termes, dans ces essais, la même inhibition est obtenue avec une concentration en peptide selon l'invention de 5 à 7 fois inférieure à la concentration en peptide non muté.

De tels résultats permettent donc d'obtenir une inhibition 5 à 7 fois supérieure avec la même concentration en peptide, ou au contraire d'obtenir la même inhibition mais avec une concentration en peptide 5 à 7 fois inférieure.

Ceci illustre l'activité inhibitrice considérablement supérieure des peptides PF-4 selon l'invention.

## Revendications

1. Peptide choisi parmi le groupe comprenant le PF4, les fragments et peptides de fusion dérivés du PF4, et leurs analogues, **caractérisé en ce que** la glutamine se situant en position 56 dans le PF4 natif est remplacée par un acide aminé basique et que ledit peptide présente une activité inhibitrice de l'angiogénèse plus importante que celle du PF4 natif.

2. Peptide selon la revendication 1, dans lequel l'acide aminé basique est l'arginine, la lysine ou l'histidine, de préférence l'arginine.

3. Peptide selon l'une ou l'autre des revendications 1 et 2, correspondant au fragment 47-70 du PF4 natif (SEQ. ID. NO :3).

4. Peptide selon l'une ou l'autre des revendications 1 et 2, correspondant au peptide de fusion 17-34/47-70 (SEQ. ID. NO :4).

5. Peptide selon l'une quelconque des revendications 1 à 4, **caractérisé en ce qu'**il présente une concentration en peptide permettant d'obtenir une inhibition de 50% de la liaison de FGF-2 aux récepteurs de haute affinité, de la liaison du VEGF aux récepteurs de surface, de la liaison du FGF-2 aux protéoglycanes ou de l'activité biologique du FGF-2 (I₅₀) inférieure à l'I₅₀ du même peptide ne présentant pas la mutation en 56.

6. Peptide selon la revendication 5, **caractérisé en ce qu'**il présente une I₅₀ de 2 à 20 fois inférieure, de préférence de 4 à 15 fois inférieure, de préférence encore de 5 à 10 fois inférieure, à l'I₅₀ du même peptide ne présentant pas la mutation en 56.

7. Peptide selon l'une quelconque des revendications 1 à 6, **caractérisé en ce qu'**il est obtenu par clivage enzymatique d'un PF-4 natif, par synthèse chimique, par des techniques recombinantes, ou par chimie combinatoire.

8. Séquence d'ADN ou ADnc ou ARNm codant un peptide selon l'une quelconque des revendications 1 à 7.

9. Médicament comprenant un peptide selon l'une quelconque des revendications 1 à 7.

10. Médicament selon la revendication 9, pour le traitement ou la prévention des maladies liées à l'angiogénèse.

11. Utilisation d'un peptide selon l'une quelconque des revendications 1 à 7 ou d'une séquence d'ADN ou ADNc ou ARNm selon la revendication 8 pour la préparation d'un médicament pour le traitement ou la prévention des maladies liées à l'angiogénèse, telles que le cancer, les maladies vasculaires de la rétine (rétinopathies), les maladies inflammatoires chroniques (comme la polyarthrite rhumatoide chronique), les angiomes, les hémangiomes et certaines hémopathies (leucémies).

## Claims

1. Peptide selected from the group consisting of PF-4, fragments and fusion peptides derived from PF-4, and their analogues, in which the glutamine situated in position 56 in the native PF-4 is replaced by a basic amino acid, said peptide having an increased angiogenesis-inhibiting activity compared to native PF-4.

2. The peptide as claimed in claim **1,** in which the basic amino acid is arginine, lysine or histidine, preferably arginine.

3. The peptide as claimed in claim **1** or **2,** corresponding to fragment 47-70 of the native PF-4 (SEQ ID NO: 3).

4. The peptide as claimed in claim **1** or **2,** corresponding to the fusion peptide 17-34/47-70 (SEQ ID NO: 4).

5. The peptide as claimed in anyone of the claims **1** to **4,** having a peptide concentration allowing to obtain a 50% inhibition of the binding of FGF-2 to high affinity receptors, of the binding of VEGF to surface receptors, of the binding of FGF-2 to proteoglycans or of the biological activity of FGF-2 (I₅₀) less than the I₅₀ of the same peptide not having the mutation in 56.

6. The peptide as claimed in claim **5,** having an I₅₀ 2 to 20 times less, preferably 4 to 15 times less, and more preferably 5 to 10 times less, than the I₅₀ of the same peptide not having the mutation in 56.

7. The peptide as claimed in anyone of the claims **1** to **6,** obtained by enzymatic cleavage of a native PF-4, by chemical synthesis, by recombinant techniques, or by combinatorial chemistry.

8. DNA or cDNA or mRNA sequence coding for a peptide as claimed in anyone of the claims **1** to **7.**

9. Medicament comprising a peptide as claimed in anyone of the claims **1** to **7.**

10. Medicament according to claim **9,** for the treatment or prevention of angiogenesis-related diseases.

11. Use of a peptide according to anyone of the claims **1** to **7** or a DNA or cDNA or mRNA sequence according to claim 8, for the preparation of a medicament for the treatment or prevention of angiogenesis-related diseases, such as cancer, vascular diseases of the retina (retinopathies), chronic inflammatory diseases (chronic rheumatoid arthritis), angiomas, hemangiomas and certain hemopathies (leukemias).

## Patentansprüche

1. Peptid ausgewählt aus der Gruppe bestehend aus PF4, den Fragmenten und Fusionspeptiden, die von TF4 abgeleitet sind und deren Analoga, **dadurch gekennzeichnet, dass** das Glutamin, das sich im nativen PF4 an Position 56 befindet, durch eine basische Aminosäure ersetzt ist und dass dieses Peptid eine die Angiogenese inhibierende Aktivität zeigt, die bedeutender ist, als die des nativen PF4.

2. Peptid gemäß Anspruch 1, in dem die basische Aminosäure Arginin, Lysin oder Histidin ist, vorzugsweise Arginin.

3. Peptid gemäß einem der Ansprüche 1 und 2, das dem Fragment 47-70 des nativen PF4 (SEQ. ID Nr. 3) entspricht.

4. Peptid gemäß einem der Ansprüche 1 und 2, das dem Fusionspeptid 17-34/47-70 (SEQ. ID Nr. 4) entspricht.

5. Peptid gemäß einem der Ansprüche 1 bis 4, **dadurch gekennzeichnet, dass** es in einer Peptidkonzentration vorliegt, die es gestattet, eine 50%ige Inhibierung der Bindung von FGF-2 an hochaffine Rezeptoren, der Bindung von VEGF an Oberflächenrezeptoren, der Bindung von FGF-2 an Proteoglykane oder der biologischen Aktivität von FGF-2 (I₅₀-Wert), die geringer ist als der I₅₀-Wert des gleichen Peptids, das nicht die Mutation an Position 56 aufweist, zu erhalten.

6. Peptid gemäß Anspruch 5, **dadurch gekennzeichnet, dass** es einen I₅₀-Wert aufweist, der 2 bis 20 mal geringer, vorzugsweise 4 bis 15 mal geringer, weiter bevorzugt 5 bis 10 mal geringer, ist als der I₅₀-Wert des gleichen Peptids, das nicht die Mutation an Position 56 aufweist.

7. Peptid gemäß einem der Ansprüche 1 bis 6, **dadurch gekennzeichnet, dass** es durch enzymatische Spaltung eines nativen PF4-Peptids, durch chemische Synthese, durch Rekombinationstechniken oder durch kombinatorische Chemie erhalten wird.

8. DNA- oder cDNA- oder mRNA-Sequenz, die für ein Peptid gemäß einem der Ansprüche 1 bis 7 codiert.

9. Medikament, das ein Peptid gemäß einem der Ansprüche 1 bis 7 umfasst.

10. Medikament gemäß Anspruch 9 zur Behandlung oder Prävention von Erkrankungen, die mit der Angiogenese in Verbindung stehen.

11. Verwendung eines Peptids gemäß einem der Ansprüche 1 bis 7 oder einer DNA- oder cDNA- oder mRNA-Sequenz gemäß Anspruch 8 zur Herstellung eines Medikaments zur Behandlung oder Prävention von Erkrankungen, die mit der Angiogenese in Verbindung stehen, wie Krebs, Gefäßerkrankungen der Retina (Retinopathien), chronische Entzündungserkrankungen (wie chronischer rheumatischer Polyarthritis), Angiome, Hämangiome und gewisse Hämopathien (Leukämien).


### LISTE DE SEQUENCES

<110> UNIVERSITE DE BORDEAUX 1
   INSTITUT DES VAISSEAUX ET DU SANG
<120> PF-4 muté, ses fragments et peptides de fusion mutés, leurs analogues, les séquences d'ADN, ADNc et ARNm correspondantes, et leur utilisation dans l'inhibition de l'angiogenèse.
<130> PF-4
<140>
   <141>
<150> FR0009464
   <151> 2000-07-19
<160> 4
<170> PatentIn Ver. 2.1
<210> 1
   <211> 70
   <212> PRT
   <213> Séquence artificielle
<220>
   <223> Description de la séquence artificielle: SEQ. ID. No : 1
<400> 1
   Glu Ala Glu Glu Asp Gly Asp Leu Gln Cys Leu Cys Val Lys Thr Thr
     1               5                  10                  15
   Ser Gln Val Arg Pro Arg His Ile Thr Ser Leu Glu Val Ile Lys Ala
                20                  25                  30
   Gly Pro His Cys Pro Thr Ala Gln Leu Ile Ala Thr Leu Lys Asn Gly
            35                  40                  45
   Arg Lys Ile Cys Leu Asp Leu Gln Ala Pro Leu Tyr Lys Lys Ile Ile
        50                  55                  60
   Lys Lys Leu Leu Glu Ser
    65                  70
<210> 2
   <211> 70
   <212> PRT
   <213> Séquence artificielle
<220>
   <223> Description de la séquence artificielle: SEQ. ID. No : 2
<400> 2
   Glu Ala Glu Glu Asp Gly Asp Leu Gln Cys Leu Cys Val Lys Thr Thr
    1               5                  10                  15
   Ser Gln Val Arg Pro Arg His Ile Thr Ser Leu Glu Val Ile Lys Ala
               20                  25                  30
   Gly Pro His Cys Pro Thr Ala Gln Leu Ile Ala Thr Leu Lys Asn Gly
           35                  40                  45
   Arg Lys Ile Cys Leu Asp Leu Arg Ala Pro Leu Tyr Lys Lys Ile Ile
       50                  55                  60
   Lys Lys Leu Leu Glu Ser
    65                  70
<210> 3
   <211> 24
   <212> PRT
   <213> Séquence artificielle
<220>
   <223> Description de la séquence artificielle: SEQ. ID. No : 3
<400> 3
   Asn Gly Arg Lys Ile Cys Leu Asp Leu Arg Ala Pro Leu Tyr Lys Lys
    1               5                  10                  15
   Ile Ile Lys Lys Leu Leu Glu Ser
               20
<210> 4
   <211> 42
   <212> PRT
   <213> Séquence artificielle
<220>
   <223> Description de la séquence artificielle: SEQ. ID. No : 4
<400> 4
   Ser Gln Val Arg Pro Arg His Ile Thr Ser Leu Glu Val Ile Lys Ala
     1               5                  10                  15
   Gly Pro Asn Gly Arg Lys Ile Cys Leu Asp Leu Arg Ala Pro Leu Tyr
               20                  25                  30
   Lys Lys Ile Ile Lys Lys Leu Leu Glu Ser
           35                  40